# EUROPÄISCHE PATENTANMELDUNG

(11) **EP 4 035 538 A1**
(43) Veröffentlichungstag der Anmeldung: **03.08.2022**
(21) Anmeldenummer: 22154298.8
(22) Anmeldetag: 31.01.2022
(51) Int. Cl.: A23L 29/238, A23L 29/30, A23L 33/105, A23L 33/15, A23L 33/16, A23L 33/175, A61K 36/23, A61K 36/81, A61K 36/39, A61K 36/882

(54) **ZUSAMMENSETZUNG**

(30) Priorität: 29.01.2021 DE 102021200826
(71) Anmelder: Süssmann, Eva, 10557 Berlin (DE)
(72) Erfinder: Süssmann, Eva, 10557 Berlin (DE)
(74) Vertreter: Patentanwälte Ruff, Wilhelm, Beier, Dauster & Partner mbB

(57) **Zusammenfassung**

Die Erfindung betrifft eine Zusammensetzung aufweisend nachfolgende Bestandteile:
- Ashwagandha oder ein Ashwagandha-Produkt,
- Shankapushpi oder ein Shankapushpi-Produkt und
- Gotu-Kola oder ein Gotu-Kola-Produkt.

Die Erfindung betrifft ferner ein Nahrungs- oder Genussmittel.

## Beschreibung

### ANWENDUNGSGEBIET UND STAND DER TECHNIK

Die Erfindung betrifft eine Zusammensetzung, insbesondere zur Verwendung als Nahrungsoder Genussmittel, vorzugsweise Getränk.

Zusammensetzungen auf Basis von Pflanzenstoffen insbesondere zur Linderung von Stress sind grundsätzlich bekannt.

Gleichwohl besteht weiterhin ein Bedarf an stresslindernden Zusammensetzungen. Insbesondere besteht ein Bedarf an Zusammensetzungen, die das Konzentrationsvermögen (sogenannter Fokus), das Entspannungsvermögen sowie den Schlaf verbessern.

### AUFGABE UND LÖSUNG

Die Erfindung stellt sich die Aufgabe, eine Zusammensetzung bereitzustellen, welche den oben genannten Bedarf adressiert. Des Weiteren stellt sich die Erfindung die Aufgabe, ein entsprechendes Nahrungs- oder Genussmittel, insbesondere Getränk, bereitzustellen.

Diese Aufgaben werden gelöst durch eine Zusammensetzung mit den Merkmalen des unabhängigen Anspruchs 1 sowie durch ein Nahrungs- oder Genussmittel gemäß Anspruch 19. Bevorzugte Ausgestaltungen der Zusammensetzung sind Gegenstand der abhängigen Ansprüche 2 bis 18. Der Wortlaut sämtlicher Ansprüche wird hiermit durch Bezugnahme ausdrücklich zum Inhalt der Beschreibung gemacht.

Gemäß einem ersten Aspekt betrifft die Erfindung eine Zusammensetzung, insbesondere eine ayurvedische Zusammensetzung, die nachfolgende Bestandteile aufweist:
- Ashwagandha oder ein Ashwagandha-Produkt,
- Shankapushpi oder ein Shankapushpi-Produkt und
- Gotu-Kola oder ein Gotu-Kola-Produkt.

Unter dem Ausdruck "Ashwagandha" soll im Sinne der vorliegenden Erfindung eine Pflanze mit dem lateinischen Namen *Withania somnifera* als solche und/oder Teile dieser Pflanze, insbesondere ausgewählt aus der Gruppe bestehend aus Wurzeln, Rinde, Blättern, Stängel, Blüten, Samen, Früchte, Beeren und Mischungen von mindestens zwei der vorgenannten Pflanzenteile, verstanden werden. Die Pflanze Ashwagandha ist auch unter der Bezeichnung Schlafbeere, Winterkirsche oder seltener unter der Bezeichnung indischer Ginseng bekannt.

Unter dem Ausdruck "Ashwagandha-Podukt" soll im Sinne der vorliegenden Erfindung ein aus der Pflanze Ashwagandha oder Teilen, insbesondere Wurzeln, Rinde, Blättern, Stängel, Blüten, Samen, Früchte, Beeren oder Mischungen, davon hergestelltes Produkt, insbesondere in Form eines Extraktes (vorzugsweise adaptogenhaltigen Extraktes), eines Pulvers (vorzugsweise adaptogenhaltigen Pulvers), eines isolierten Adaptogens oder einer isolierten Adaptogenmischung, oder ein synthetisiertes, insbesondere chemisch synthetisiertes, Adaptogen, das natürlicherweise in Ashwagandha vorkommt, oder eine synthetisierte, insbesondere chemisch synthetisierte, Adaptogenmischung, die natürlicherweise in Ashwagandha vorkommt, verstanden werden.

Unter dem Ausdruck "Adaptogen" soll im Sinne der vorliegenden Erfindung ein biologisch aktiver Pflanzenstoff verstanden werden, der dem Organismus helfen soll, sich erhöhten körperlichen und emotionalen Stresssituationen anzupassen.

Unter dem Ausdruck "Shankapushpi" soll im Sinne der vorliegenden Erfindung eine Pflanze mit dem lateinischen Namen *Convolvulus pluricaulis* als solche und/oder Teile dieser Pflanze, insbesondere ausgewählt aus der Gruppe bestehend aus Wurzeln, Rinde, Blättern, Stängel, Blüten, Samen, Früchte, Beeren und Mischungen von mindestens zwei der vorgenannten Pflanzenteile, verstanden werden.

Unter dem Ausdruck "Shankapushpi-Produkt" soll im Sinne der vorliegenden Erfindung ein aus der Pflanze Shankapushpi oder Teilen, insbesondere Wurzeln, Rinde, Blättern, Stängel, Blüten, Samen, Früchte, Beeren oder Mischungen, davon hergestelltes Produkt, insbesondere in Form eines Extraktes (vorzugsweise adaptogenhaltigen Extraktes), eines Pulvers (vorzugsweise adaptogenhaltigen Pulvers), eines isolierten Adaptogens oder einer isolierten Adaptogenmischung, oder ein synthetisiertes, insbesondere chemisch synthetisiertes, Adaptogen, das natürlicherweise in Shankapushpi vorkommt, oder eine synthetisierte, insbesondere chemisch synthetisierte, Adaptogenmischung, die natürlicherweise in Shankapushpi vorkommt, verstanden werden.

Unter dem Ausdruck "Gotu-Kola" soll im Sinne der vorliegenden Erfindung eine Pflanze mit dem lateinischen Namen *Centella asiatica* als solche und/oder Teile dieser Pflanze, insbesondere ausgewählt aus der Gruppe bestehend aus Wurzeln, Rinde, Blättern, Stängel, Blüten, Samen, Früchte, Beeren und Mischungen von mindestens zwei der vorgenannten Pflanzenteile, verstanden werden. Die Pflanze Gotu-Kola ist auch unter der Bezeichnung indischer Wassernabel, asiatischer Wassernabel oder Tigergras bekannt.

Unter dem Ausdruck "Gotu-Kola-Produkt" soll im Sinne der vorliegenden Erfindung ein aus der Pflanze Gotu-Kola oder Teilen, insbesondere Wurzeln, Rinde, Blättern, Stängel, Blüten, Samen, Früchte, Beeren oder Mischungen, davon hergestelltes Produkt, insbesondere in Form eines Extraktes (vorzugsweise adaptogenhaltigen Extraktes), eines Pulvers (vorzugsweise adaptogenhaltigen Pulvers), eines isolierten Adaptogens oder einer isolierten Adaptogenmischung, oder ein synthetisiertes, insbesondere chemisch synthetisiertes, Adaptogen, das natürlicherweise in Gotu-Kola vorkommt, oder eine synthetisierte, insbesondere chemisch synthetisierte, Adaptogenmischung, die natürlicherweise in Gotu-Kola vorkommt, verstanden werden.

Unter dem Ausdruck "Extrakt" soll im Sinne der vorliegenden Erfindung vorzugsweise eine konzentrierte, bevorzugt auf einen bestimmten Wirkstoffgehalt, insbesondere Adaptogengehalt, eingestellte Zubereitung, aufweisend oder bestehend aus Wirkstoffen, insbesondere Adaptogenen, verstanden werden. Bei dem Extrakt kann es sich insbesondere um einen alkoholischen Extrakt handeln. Der alkoholische Extrakt kann direkt als Flüssigextrakt oder nach Entfernen des Auszugs- oder Extraktionsmittels als Trockenextrakt vorliegen. Beispielsweise kann es sich bei dem Extrakt um einen alkoholischen Extrakt mit einem Ethanolgehalt von ≥ 30 Gew.-% und einem Wassergehalt von ≤ 70 Gew.-%, bezogen auf das Gesamtgewicht des Extraktionsmittels, handeln. Zum Beispiel kann der alkoholische Extrakt einen Anteil an Ethanol von ≥ 40 Gew.-%, insbesondere ≥ 60 Gew.-%, insbesondere ≥ 80 Gew.-%, insbesondere ≥ 90 Gew.-%, bezogen auf das Gesamtgewicht des Extraktionsmittels, aufweisen, wobei der Rest Wasser ist.

Die Erfinderin konnte überraschenderweise zeigen, dass die erfindungsgemäße Zusammensetzung mit den vorgenannten Bestandteilen in der Lage ist, Stress, insbesondere emotionalen Stress und/oder körperlichen Stress, zu verringern.

In Ausgestaltung der Erfindung weist die Zusammensetzung ferner nachfolgenden Bestandteil auf:
- Theanin (N-Ethyl-glutamin), insbesondere L-Theanin (N-Ethyl-L-glutamin).

Durch den zusätzlichen Bestandteil Theanin, insbesondere L-Theanin, lässt sich die Wirkung der Zusammensetzung, Stress, insbesondere emotionalen Stress und/oder körperlichen Stress, zu verringern, vorteilhafter Weise zusätzlich optimieren oder verstärken.

In weiterer Ausgestaltung der Erfindung weist die Zusammensetzung pro Darreichungsportion oder Darreichungsform 50 mg bis 1000 mg, insbesondere 380 mg bis 1000 mg, von dem Bestandteil Theanin, insbesondere L-Theanin, auf.

Unter dem Ausdruck "Darreichungsportion" soll im Sinne der vorliegenden Erfindung eine für eine Darreichung oder Verabreichung vorgesehene Portion der Zusammensetzung oder eines die Zusammensetzung aufweisenden Nahrungs- oder Genussmittels, insbesondere Getränks, verstanden werden. Liegt die Zusammensetzung beispielsweise in flüssiger Form vor oder ist die Zusammensetzung beispielsweise Bestandteil eines Getränks, kann eine geeignete Darreichungsportion oder -form beispielsweise ein Flüssigkeitsvolumen von 25 ml, 30 ml, 50 ml, 100 ml, 150 ml, 200 ml, 250 ml, 500 ml, 750 ml oder 1000 ml aufweisen. Liegt die Zusammensetzung dagegen beispielsweise in fester Form vor oder ist die Zusammensetzung beispielsweise Bestandteil eines in fester Form vorliegenden Nahrungs- oder Genussmittels, kann eine geeignete Darreichungsportion beispielsweise eine Masse von 25 g, 30 g, 50 g, 100 g, 150 g, 200 g, 250 g, 500 g, 750 g oder 1 kg aufweisen.

Insbesondere kann das Theanin, insbesondere L-Theanin, bevorzugt einen Anteil von 0,1 Gew.-% bis 6 Gew.-%, insbesondere 0,3 Gew.-% bis 3 Gew.-%, vorzugsweise 0,5 Gew.-% bis 2 Gew.-%, aufweisen, bezogen auf das Gesamtgewicht der Zusammensetzung. Liegt die Zusammensetzung als Feststoff, insbesondere als Pulver, vor, kann ein Anteil von Theanin, insbesondere L-Theanin, von 0,1 Gew.-% bis 6 Gew.-%, bezogen auf das Gesamtgewicht der Zusammensetzung, bevorzugt sein. Liegt die Zusammensetzung dagegen in flüssiger Form vor, kann ein Anteil von Theanin, insbesondere L-Theanin, von 0,1 Gew.-% bis 0,4 Gew.-%, bezogen auf das Gesamtgewicht der Zusammensetzung, bevorzugt sein.

In weiterer Ausgestaltung der Erfindung handelt es sich bei dem Ashwagandha-Produkt um einen Extrakt, insbesondere Wurzel-, Rinden-, Blätter-, Stängel-, Blüten-, Samen-, Früchteoder Beerenextrakt, von Ashwagandha. Bevorzugt liegt der Extrakt, insbesondere Wurzel-, Rinden-, Blätter-, Stängel-, Blüten-, Samen-, Früchte- oder Beerenextrakt, von Ashwagandha in Form eines Pulvers vor. Weiter bevorzugt handelt es sich bei dem Extrakt, insbesondere Wurzel-, Rinden-, Blätter-, Stängel-, Blüten-, Samen-, Früchte- oder Beerenextrakt, von Ashwaganda um einen 14:1 Trockenextrakt.

Erfindungsgemäß ist es insbesondere bevorzugt, wenn es sich bei dem Ashwaganda-Produkt um einen Wurzelextrakt von Ashwaganda handelt.

Der Extrakt, insbesondere Wurzel-, Rinden-, Blätter-, Stängel-, Blüten-, Samen-, Früchte- oder Beerenextrakt, von Ashwaganda wird vorzugsweise hergestellt, in dem Ashwaganda oder Teile, insbesondere Wurzeln, Blätter, Stängel, Blüten, Samen, Früchte oder Beeren, davon mit Wasser, insbesondere ausschließlich mit Wasser, oder einem Gemisch aus Wasser und einem Alkohol, wie beispielsweise Methanol und/oder Ethanol, extrahiert wird/werden. Bevorzugt wird der Extrakt, insbesondere Wurzel-, Rinde-, Blätter-, Stängel-, Blüten-, Samen-, Früchte- oder Beerenextrakt, nach der Extraktion bei Unterdruck oder Vakuum konzentriert. Zum Entfernen von Withanolid A-Aglycone kann es ferner vorgesehen sein, das Konzentrat anschließend mit einem apolaren organischen Lösungsmittel, wie beispielsweise Chloroform und/oder Ethylacetat, zu behandeln. Danach kann sich eine Trocknung, insbesondere Vakuumtrocknung, des Extrakts, insbesondere des so behandelten Extrakts, insbesondere unterhalb von 60 °C, anschließen. Hierbei wird vorzugsweise ein Feststoff, insbesondere trockener Feststoff, erhalten. Der Feststoff kann anschließend, insbesondere unter kontrollierten Temperatur- und Feuchtigkeitsbedingungen, pulverisiert werden. Die Herstellung eines solchen Ashwaganda-Extrakts ist beispielsweise in der US 6,153,198 beschrieben, deren Offenbarungsgehalt insoweit durch ausdrückliche Bezugnahme zum Inhalt der vorliegenden Beschreibung gemacht wird. Bezüglich weiterer geeigneter Verfahren zur Herstellung von Ashwagandha-Extrakten wird ferner auf das Hagers Handbuch (Band 2, Springerverlag 1991, Seiten 1024 - 1031) Bezug genommen, dessen Offenbarungsgehalt bezüglich der dort beschriebenen Verfahren zur Herstellung von Pflanzen- bzw. Drogenextrakten durch ausdrückliche Bezugnahme ebenfalls zum Inhalt der vorliegenden Beschreibung gemacht wird.

In weiterer Ausgestaltung der Erfindung weist der Extrakt, insbesondere Wurzel-, Rinden-, Blätter-, Stängel-, Blüten-, Samen-, Früchte- oder Beerenextrakt, von Ashwaganda mindestens einen Inhaltsstoff, insbesondere mindestens ein Adaptogen, vorzugsweise ausgewählt aus der Gruppe bestehend aus Withanolide, Withanolidglykoside, Saponide, Sitoindoside, Alkaloide, Steroidlactone, Tropanole, Cuscohygren, Choline, Fettsäuren, Oligosaccharide, insbesondere Oligosaccharide mit einem relativen Molekulargewicht < 2000, Aminosäuren und Mischungen von mindestens zwei der vorgenannten Inhaltsstoffe, insbesondere Adaptogene, auf.

Bevorzugt handelt es sich bei den Whitanoliden um Whitanolid A, Withanon, Withaferin A, Desoxywithaferin, Somniwithaloid, Somniferanold, Salpichrolid A, Nicandrenon-1, Ixocarpalacton A oder eine Mischung von mindestens zwei der vorgenannten Whitanolide.

Bei den Steroidlactonen kann es sich insbesondere um C-28-Steroidlactone handeln. Insbesondere können die C-28-Steroidlactone ein Grunderüst mit vier Cycloalkanringen oder drei Cyclohexanringen und einem Cyclopentanring aufweisen.

Bei den Aminosäuren kann es sich insbesondere um Tryptophan, Tyrosin, Alanin, Glycin, Aspartat oder um Mischungen von mindestens zwei der vorgenannten Aminosäuren handeln.

In weiterer Ausgestaltung der Erfindung weist die Zusammensetzung pro Darreichungsportion oder Darreichungsform 50 mg bis 400 mg, insbesondere 50 mg bis 300 mg, von dem Bestandteil Ashwaganda oder Ashwaganda-Produkt auf.

Insbesondere kann der Bestandteil Ashwagandha oder Ashwagandha-Produkt bevorzugt einen Anteil von 0,1 Gew.-% bis 2 Gew.-%, bevorzugt 0,1 Gew.-% bis 1,7 Gew.-%, insbesondere 0,1 Gew.-% bis 0,6 Gew.-%, aufweisen, bezogen auf das Gesamtgewicht der Zusammensetzung. Liegt die Zusammensetzung als Feststoff, insbesondere als Pulver, vor, kann ein Anteil von Ashwagandha oder des Ashwagandha-Produkts von 0,1 Gew.-% bis 2 Gew.-%, bezogen auf das Gesamtgewicht der Zusammensetzung, bevorzugt sein. Liegt die Zusammensetzung dagegen in flüssiger Form vor, kann ein Anteil von Ashwagandha oder des Ashwagandha-Produkts von 0,1 Gew.-% bis 0,2 Gew.-%, bezogen auf das Gesamtgewicht der Zusammensetzung, bevorzugt sein.

In weiterer Ausgestaltung der Erfindung handelt es sich bei dem Shankapushpi-Produkt um einen Extrakt, insbesondere Wurzel-, Rinden-, Blätter-, Stängel-, Blüten-, Samen-, Früchteoder Beerenextrakt, von Shankapushpi. Bevorzugt liegt der Extrakt, insbesondere Wurzel-, Rinden-, Blätter-, Stängel-, Blüten-, Samen-, Früchte- oder Beerenextrakt, von Shankapushpi in Form eines Pulvers vor. Weiter bevorzugt handelt es sich bei dem Extrakt, insbesondere Wurzel-, Rinden-, Blätter-, Stängel-, Blüten-, Samen-, Früchte- oder Beerenextrakt, von Shankapushpi um einen 4:1 Trockenextrakt.

Der Extrakt, insbesondere Wurzel-, Rinden-, Blätter-, Stängel-, Blüten-, Samen-, Früchte- oder Beerenextrakt, von Shankapushpi kann insbesondere hergestellt werden, in dem Shankapushpi oder Teile, insbesondere Wurzeln, Blätter, Stängel, Blüten, Samen, Früchte oder Beeren, davon mit Wasser, insbesondere ausschließlich mit Wasser, oder einem Gemisch aus Wasser und einem Alkohol, wie beispielsweise Methanol und/oder Ethanol, extrahiert wird/werden. Beispielsweise kann der Extrakt, insbesondere Wurzel-, Rinde-, Blätter-, Stängel-, Blüten-, Samen-, Früchte- oder Beerenextrakt, nach der Extraktion bei Unterdruck oder Vakuum konzentriert werden. Danach kann sich eine Trocknung, insbesondere Vakuumtrocknung, des Extrakts, insbesondere konzentrierten Extrakts, anschließen. Hierbei wird vorzugsweise ein Feststoff, insbesondere trockener Feststoff, erhalten. Der Feststoff kann anschließend, insbesondere unter kontrollierten Temperatur- und Feuchtigkeitsbedingungen, pulverisiert werden. Bezüglich weiterer geeigneter Verfahren zur Herstellung von Shankapushpi-Extrakten wird auf das Hagers Handbuch (Band 2, Springerverlag 1991, Seiten 1024 - 1031) Bezug genommen, dessen Offenbarungsgehalt bezüglich der dort beschriebenen Verfahren zur Herstellung von Pflanzen- bzw. Drogenextrakten durch ausdrückliche Bezugnahme zum Inhalt der vorliegenden Beschreibung gemacht wird.

In weiterer Ausgestaltung der Erfindung weist der Extrakt, insbesondere Wurzel-, Rinden-, Blätter-, Stängel-, Blüten-, Samen-, Früchte- oder Beerenextrakt, von Shankapushpi mindestens einen Inhaltsstoff, insbesondere mindestens ein Adaptogen, vorzugsweise ausgewählt aus der Gruppe bestehend aus Kämpferol, β-Sitosterol, N-Hexacosanol, Hydroxyzimtsäure und Mischungen von mindestens zwei der vorgenannten Inhaltsstoffe, insbesondere Adaptogene, auf.

In weiterer Ausgestaltung der Erfindung weist die Zusammensetzung pro Darreichungsportion oder Darreichungsform 100 mg bis 600 mg, insbesondere 150 mg bis 500 mg, von dem Bestandteil Shankapushpi oder Shankapushpi-Produkt auf.

Insbesondere kann der Bestandteil Shankapushpi oder das Shankapushpi-Produkt einen Anteil von 0,2 Gew.-% bis 5 Gew.-%, insbesondere 0,2 Gew.-% bis 1 Gew.-%, vorzugsweise 0,3 Gew.-% bis 1 Gew.-%, aufweisen, bezogen auf das Gesamtgewicht der Zusammensetzung. Liegt die Zusammensetzung als Feststoff, insbesondere als Pulver, vor, kann ein Anteil von Shankapushpi oder des Shankapushpi-Produkts von 0,1 Gew.-% bis 5 Gew.-%, bezogen auf das Gesamtgewicht der Zusammensetzung, bevorzugt sein. Liegt die Zusammensetzung dagegen in flüssiger Form vor, kann ein Anteil von Shankapushpi oder des Shankapushpi-Produkts von 0,2 Gew.-% bis 0,3 Gew.-%, bezogen auf das Gesamtgewicht der Zusammensetzung, bevorzugt sein.

In weiterer Ausgestaltung der Erfindung handelt es sich bei dem Gotu-Kola-Produkt um einen Extrakt, insbesondere Wurzel-, Rinden-, Blätter-, Stängel-, Blüten-, Samen-, Früchte- oder Beerenextrakt, von Gotu-Kola. Bevorzugt liegt der Extrakt, insbesondere Wurzel-, Rinden-, Blätter-, Stängel-, Blüten-, Samen-, Früchte- oder Beerenextrakt, in Form eines Pulvers vor. Weiter bevorzugt handelt es sich bei dem Extrakt, insbesondere Wurzel-, Rinden-, Blätter-, Stängel-, Blüten-, Samen-, Früchte- oder Beerenextrakt, von Gotu-Kola um einen 6:1 Trockenextrakt.

Der Extrakt von Gotu-Kola kann beispielsweise nach der Vorschrift 4a HAB (Homöopathisches Arzneibuch 2001) durch Extraktion von Gotu-Kola mit 62 Gew.-% Ethanol und 38 Gew.-% Wasser hergestellt werden. Bevorzugt werden für die Extraktion getrocknete oberirdische Teile von Gotu-Kola verwendet. Bezüglich weiterer geeigneter Verfahren zur Extraktion von Gotu-Kola wird auf das Hagers Handbuch (Band 2, Springerverlag 1991, Seiten 1024 - 1031) Bezug genommen, dessen Offenbarungsgehalt bezüglich der dort beschriebenen Verfahren zur Herstellung von Pflanzen- bzw. Drogenextrakten durch ausdrückliche Bezugnahme zum Inhalt der vorliegenden Beschreibung gemacht wird. In weiterer Ausgestaltung der Erfindung weist der Extrakt, insbesondere Wurzel-, Rinden-, Blätter-, Stängel-, Blüten-, Samen-, Früchte- oder Beerenextrakt, von Gotu-Kola mindestens einen Inhaltsstoff, insbesondere mindestens ein Adaptogen, vorzugsweise ausgewählt aus der Gruppe bestehend aus Triterpenoide, insbesondere Asiaticoside, Asiatsäure oder Madecassische Säure, Flavonolglykoside, Alkaloide, insbesondere Hydrocotylin, Caryophyllen, Cymen, Germacren, Pinen und Mischungen von mindestens zwei der vorgenannten Inhaltsstoffe, insbesondere Adaptogene, auf.

In weiterer Ausgestaltung der Erfindung weist die Zusammensetzung pro Darreichungsportion oder Darreichungsform 50 mg bis 300 mg, insbesondere 100 mg bis 200 mg, von dem Bestandteil Gotu-Kola oder Gotu-Kola-Produkt auf.

Insbesondere kann der Bestandteil Gotu-Kola oder das Gotu-Kola-Produkt einen Anteil von 0,1 Gew.-% bis 3,5 Gew.-%, bevorzugt 0,2 Gew.-% bis 3,5 Gew.-%, insbesondere 0,2 Gew.-% bis 0,4 Gew.-%, aufweisen, bezogen auf das Gesamtgewicht der Zusammensetzung. Liegt die Zusammensetzung als Feststoff, insbesondere als Pulver, vor, kann ein Anteil von Gotu-Kola oder des Gotu-Kola-Produkts von 0,1 Gew.-% bis 3,5 Gew.-%, bezogen auf das Gesamtgewicht der Zusammensetzung, bevorzugt sein. Liegt die Zusammensetzung dagegen in flüssiger Form vor, kann ein Anteil von Gotu-Kola oder des Gotu-Kola-Produkts von 0,2 Gew.-% bis 0,3 Gew.-%, bezogen auf das Gesamtgewicht der Zusammensetzung, bevorzugt sein.

In weiterer Ausgestaltung der Erfindung weist die Zusammensetzung ferner nachfolgende Bestandteile auf:
- Kalmus oder ein Kalmus-Produkt
   und
- Shilajit oder ein Shilajit-Produkt oder
- Klamath-Algen oder ein Klamath-Algen-Produkt,
- Kalmus oder ein Kalmus-Produkt
   und
- Shilajit oder ein Shilajit-Produkt.

Die Erfindung beruht auf der weiteren überraschenden Erkenntnis, dass eine solche Zusammensetzung das individuelle Entspannungsvermögen spürbar verbessert.

Unter dem Ausdruck "Klamath-Algen" sollen im Sinne der vorliegenden Erfindung Cyanobakterien mit dem lateinischen Namen *Aphanizomenon flos-aquae* als solche und/oder Bestandteile davon verstanden werden. Die Klamath-Algen sind auch unter der Bezeichnung grüne Spanalgen, AFA-Algen oder "blaugrüne Algen" bekannt.

Bevorzugt weist die Zusammensetzung pro Darreichungsportion oder Darreichungsform 500 mg bis 1000 mg, insbesondere 600 mg bis 800 mg, von dem Bestandteil Klamath-Algen oder Klamath-Algen-Produkt auf.

Insbesondere kann der Bestandteil Klamath-Algen oder Klamath-Algen-Produkt einen Anteil von 1 Gew.-% bis 2 Gew.-%, insbesondere 1 Gew.-% bis 1,8 Gew.-%, vorzugsweise 1,2 Gew.- % bis 1,6 Gew.-%, aufweisen, bezogen auf das Gesamtgewicht der Zusammensetzung.

Weiter bevorzugt handelt es sich bei dem Klamath-Algen-Produkt um mindestens einen Wirkstoff der Klamath-Algen. Alternativ handelt es sich bei dem Klamath-Algen-Produkt vorzugsweise um einen Extrakt aus den Klamath-Algen, wobei der Extrakt mindestens einen Wirkstoff der Klamath-Algen aufweist oder aus mindestens einem Wirkstoff der Klamath-Algen besteht. Insbesondere kann es sich bei dem Klamath-Algen-Produkt um ein Pulver, insbesondere einen Pulverextrakt, aus den Klamath-Algen handeln, wobei das Pulver, insbesondere der Pulverextrakt, mindestens einen Wirkstoff der Klamath-Algen aufweist oder aus mindestens einem Wirkstoff der Klamath-Algen besteht.

Unter dem Ausdruck "Kalmus" soll im Sinne der vorliegenden Erfindung eine Pflanze mit dem lateinischen Namen *Acorus calamus* als solche und/oder Teile dieser Pflanze, insbesondere ausgewählt aus der Gruppe bestehend aus Wurzeln, Rinde, Blättern, Stängel, Blüten, Samen, Früchte, Beeren und Mischungen von mindestens zwei der vorgenannten Pflanzenteile, verstanden werden. Die Pflanze Kalmus ist auch unter der Bezeichnung indischer Kalmus bekannt.

Unter dem Ausdruck "Kalmus-Produkt" soll im Sinne der vorliegenden Erfindung ein aus der Pflanze Kalmus oder Teilen, insbesondere Wurzeln, Rinde, Blättern, Stängel, Blüten, Samen, Früchte, Beeren oder Mischungen, davon hergestelltes Produkt, insbesondere in Form eines Extraktes (vorzugsweise adaptogenhaltigen Extraktes), eines eines Pulvers (vorzugsweise adaptogenhaltigen Pulvers), eines isolierten Adaptogens oder einer isolierten Adaptogenmischung, oder ein synthetisiertes, insbesondere chemisch synthetisiertes, Adaptogen, das natürlicherweise in Kalmus vorkommt, oder eine synthetisierte, insbesondere chemisch synthetisierte, Adaptogenmischung, die natürlicherweise in Kalmus vorkommt, verstanden werden.

Bevorzugt handelt es sich bei dem Kalmus-Produkt um einen Extrakt, insbesondere Wurzel-, Rinden-, Blätter-, Stängel-, Blüten-, Samen-, Früchte- oder Beerenextrakt, von Kalmus. Weiter bevorzugt handelt es sich bei dem Extrakt, insbesondere Wurzel-, Rinden-, Blätter-, Stängel-, Blüten-, Samen-, Früchte- oder Beerenextrakt, von Kalmus um einen 4:1 Extrakt.

Weiter bevorzugt weist die Zusammensetzung pro Darreichungsportion oder Darreichungsform 10 mg bis 100 mg, insbesondere 40 mg bis 60 mg, von dem Bestandteil Kalmus oder Kalmus-Produkt auf.

Insbesondere kann der Bestandteil Kalmus oder Kalmus-Produkt einen Anteil von 0,02 Gew.-% bis 0,2 Gew.-%, insbesondere 0,04 Gew.-% bis 0,16 Gew.-%, vorzugsweise 0,08 Gew.-% bis 0,1 Gew.-%, aufweisen, bezogen auf das Gesamtgewicht der Zusammensetzung.

Unter dem Ausdruck "Shilajit" soll im Sinne der vorliegenden Erfindung ein hell- bis dunkelbraunes, je nach Gehalt ein pulverförmiges bis zähviskoses, asphaltartiges Naturprodukt mit arttypischem, harzig bis rauchigem Geruch (*Asphaltum punjabinum*) verstanden werden. Das Naturprodukt stellt ein hochmolekulares organisch-minerales Stoffwechselprodukt aerober Mikroorganismen, entstanden im Verwesungsprozess von Pflanzenresten, Flechten und Harzen, dar. Shilajit ist auch unter der Bezeichnung Mumijo bekannt. Der Bestandteil Shilajit kann insbesondere ausgewählt sein aus der Gruppe bestehend aus Artscha-Mumijo, Bitumen-Mumijo, Honig-Wachs-Mumijo, mineralisches Mumijo, Flechten-Mumijo und Mischungen von mindestens zwei der vorgenannten Shilajit-Arten bzw. Mumijo-Arten.

Bevorzugt weist die Zusammensetzung pro Darreichungsportion oder Darreichungsform 50 mg bis 1000 mg, insbesondere 100 mg bis 600 mg, von dem Bestandteil Shilajit oder Shilajit-Produkt auf.

Insbesondere kann der Bestandteil Shilajit oder das Shilajit-Produkt einen Anteil von 0,1 Gew.- % bis 2 Gew.-%, insbesondere 0,2 Gew.-% bis 1,2 Gew.-%, vorzugsweise 0,2 Gew.-% bis 1 Gew.-%, aufweisen, bezogen auf das Gesamtgewicht der Zusammensetzung.

Weiter bevorzugt handelt es sich bei dem Shilajit-Produkt um mindestens einen Wirkstoff von Shilajit. Alternativ handelt es sich bei dem Shilajit-Produkt vorzugsweise um einen Extrakt aus Shilajit, wobei der Extrakt mindestens einen Wirkstoff von Shilajit aufweist oder aus mindestens einem Wirkstoff von Shilajit besteht. Insbesondere kann es sich bei dem Shilajit-Produkt um ein Pulver, insbesondere einen Pulverextrakt, aus Shilajit handeln, wobei das Pulver, insbesondere der Pulverextrakt, mindestens einen Wirkstoff von Shilajit aufweist oder aus mindestens einem Wirkstoff von Shilajit besteht.

In weiterer Ausgestaltung der Erfindung weist die Zusammensetzung ferner nachfolgende Bestandteile auf:
- Koffein und/oder einen Stoff mit koffeinähnlicher Wirkung und/oder ein koffeinhaltiges Produkt
   und
- Cordyceps oder ein Cordyceps-Produkt.

Besonders bevorzugt weist die Zusammensetzung ferner nachfolgende Bestandteile auf:
- Koffein und/oder einen Stoff mit koffeinähnlicher Wirkung und/oder ein koffeinhaltiges Produkt,
- Cordyceps oder ein Cordyceps-Produkt,
- Klamath-Algen oder ein Klamath-Algen-Produkt,
- Kalmus oder ein Kalmus-Produkt
   und
- Shilajit oder ein Shilajit-Produkt.

Die Erfindung beruht auf dem weiteren überraschenden Befund, dass eine solche Zusammensetzung das individuelle Konzentrationsvermögen spürbar verbessert.

Unter dem Ausdruck "Cordyceps" soll im Sinne der vorliegenden Erfindung ein Schlauchpilz, vorzugsweise chinesischer Raupenpilz (auch als tibetischer Raupenpilz oder tibetischer Raupenkeulenpilz bekannt), d.h. ein Schlauchpilz mit dem lateinischen Namen *Ophiocordyceps sinensis* oder *Cordyceps sinensis,* und/oder Puppen-Kernkeule, d.h. ein Schlauchpilz mit dem lateinischen Namen *Cordyceps militaris,* als solcher und/oder Teile dieses Schlauchpilzes, insbesondere ausgewählt aus der Gruppe bestehend aus Fruchtkörper, Pilzgeflecht, Pilzfäden, und Mischungen von mindestens zwei der vorgenannten Schlauchpilzteile, verstanden werden.

Demnach handelt es sich bei dem oben erwähnten Bestandteil Cordyceps vorzugsweise um den chinesischen Raupenpilz und/oder die Puppen-Kernkeule.

Bei dem oben erwähnten Cordyceps-Produkt handelt es sich dementsprechend bevorzugt um ein Produkt des chinesischen Raupenpilzes und/oder um ein Produkt der Puppen-Kernkeule.

Unter dem Ausdruck "Cordyceps-Produkt", insbesondere "Produkt des chinesischen Raupenpilzes" und/oder "Produkt der Puppen-Kernkeule" soll im Sinne der vorliegenden Erfindung ein aus Cordyceps, insbesondere des chinesischen Raupenpilzes und/oder der Puppen-Kernkeule, oder Teilen davon hergestelltes Produkt, insbesondere in Form eines Extraktes (vorzugsweise wirkstoffhaltigen Extraktes), eines Pulvers (vorzugsweise wirkstoffhaltigen Pulvers), eines isolierten Wirkstoffes oder einer isolierten Wirkstoffmischung, oder ein synthetisierter, insbesondere chemisch synthetisierter, Wirkstoff, der natürlicherweise in Cordyceps, insbesondere in *Ophiocordyceps sinensis* oder *Cordyceps sinensis* und/oder *Cordyceps militaris,* vorkommt, oder eine synthetisierte, insbesondere chemisch synthetisierte, Wirkstoffmischung, die natürlicherweise in Cordyceps, insbesondere in *Ophiocordyceps sinensis* oder *Cordyceps sinensis* und/oder *Cordyceps militaris,* vorkommt, verstanden werden. Vorzugsweise liegt das Cordyceps-Produkt in Form eines Extraktes, insbesondere 20:1 Extraktes, vor.

Bevorzugt weist die Zusammensetzung pro Darreichungsportion oder Darreichungsform 20 mg bis 250 mg, insbesondere 100 mg bis 200 mg, von dem Bestandteil Koffein und/oder Stoff mit koffeinähnlicher Wirkung und/oder koffeinhaltigen Produkt auf.

Insbesondere kann der Bestandteil Koffein und/oder Stoff mit koffeinähnlicher Wirkung und/oder koffeinhaltige Produkt einen Anteil von 0,04 Gew.-% bis 0,5 Gew.-%, insbesondere 0,1 Gew.-% bis 0,5 Gew.-%, vorzugsweise 0,2 Gew.-% bis 0,4 Gew.-%, aufweisen, bezogen auf das Gesamtgewicht der Zusammensetzung.

Bei dem koffeinhaltigen Produkt kann es sich insbesondere um einen Extrakt oder ein Pulver handeln. Bevorzugt handelt es sich bei dem koffeinhaltigen Produkt um einen Extrakt aus Guarana oder um ein Pulver von Guarana. Der Extrakt kann insbesondere in Form eines Pulvers vorliegen.

Unter dem Ausdruck "Guaraná" soll im Sinne der vorliegenden Erfindung eine Pflanze mit dem lateinischen Namen *Paullinia cupana* als solche und/oder Teile dieser Pflanze, insbesondere ausgewählt aus der Gruppe bestehend aus Wurzeln, Rinde, Blättern, Stängel, Blüten, Samen, Früchte, Beeren und Mischungen von mindestens zwei der vorgenannten Pflanzenteile, verstanden werden.

Bevorzugt weist die Zusammensetzung pro Darreichungsportion oder Darreichungsform 50 mg bis 500 mg, insbesondere 100 mg bis 350 mg, von dem Bestandteil Cordyceps oder Cordyceps-Produkt auf.

Insbesondere kann der Bestandteil Cordyceps oder Cordyceps-Produkt einen Anteil von 0,1 Gew.-% bis 1 Gew.-%, insbesondere 0,2 Gew.-% bis 0,7 Gew.-%, vorzugsweise 0,2 Gew.-% bis 0,5 Gew.-%, aufweisen, bezogen auf das Gesamtgewicht der Zusammensetzung.

In weiterer Ausgestaltung der Erfindung weist die Zusammensetzung ferner nachfolgende Bestandteile auf:
- Melatonin,
- L- Tryptophan
   und
- 5-Hydroxytryptophan oder ein 5-hydroxytryptophanhaltiges Produkt.

Die Erfindung beruht auf der weiteren überraschenden Erkenntnis, dass eine solche Zusammensetzung das individuelle Schlafvermögen bzw. die individuelle Schlafqualität deutlich verbessert.

Bevorzugt weist die Zusammensetzung pro Darreichungsportion oder Darreichungsform 0,1 mg bis 2 mg, insbesondere 0,5 mg bis 1 mg, von dem Bestandteil Melatonin auf.

Insbesondere kann der Bestandteil Melatonin einen Anteil von 0,0002 Gew.-% bis 0,004 Gew.- %, insbesondere 0,0002 Gew.-% bis 0,002 Gew.-%, vorzugsweise 0,001 Gew.-% bis 0,002 Gew.-%, aufweisen, bezogen auf das Gesamtgewicht der Zusammensetzung.

Bevorzugt weist die Zusammensetzung pro Darreichungsportion oder Darreichungsform 50 mg bis 500 mg, insbesondere 150 mg bis 400 mg, von dem Bestandteil L-Tryptophan auf.

Insbesondere kann der Bestandteil L- Tryptophan einen Anteil von 0,1 Gew.-% bis 1 Gew.-%, insbesondere 0,2 Gew.-% bis 0,8 Gew.-%, vorzugsweise 0,3 Gew.-% bis 0,8 Gew.-%, aufweisen, bezogen auf das Gesamtgewicht der Zusammensetzung.

Bevorzugt weist die Zusammensetzung pro Darreichungsportion oder Darreichungsform 50 mg bis 500 mg, insbesondere 150 mg bis 300 mg, von dem Bestandteil 5-Hydroxytryptophan oder 5-hydroxytryptophanhaltiges Produkt auf.

Insbesondere kann der Bestandteil 5-Hydroxytryptophan oder 5-hydroxytryptophanhaltiges Produkt einen Anteil von 0,1 Gew.-% bis 1 Gew.-%, insbesondere 0,2 Gew.-% bis 0,8 Gew.-%, vorzugsweise 0,3 Gew.-% bis 0,6 Gew.-%, aufweisen, bezogen auf das Gesamtgewicht der Zusammensetzung.

Bevorzugt handelt es sich bei dem 5-hydroxytryptophanhaltigen Produkt um einen Extrakt aus Griffonia oder um ein Pulver von Griffonia. Der Extrakt kann insbesondere in Form eines Pulvers vorliegen.

Unter dem Ausdruck "Griffonia" soll im Sinne der vorliegenden Erfindung eine Pflanze mit dem lateinischen Namen *Griffonia simplicifolia* (auch als afrikanische Schwarzbohne bekannt) als solche und/oder Teile dieser Pflanze, insbesondere ausgewählt aus der Gruppe bestehend aus Wurzeln, Rinde, Blättern, Stängel, Blüten, Samen, Früchte, Beeren und Mischungen von mindestens zwei der vorgenannten Pflanzenteile, verstanden werden.

In weiterer Ausgestaltung der Erfindung weist die Zusammensetzung ferner mindestens einen süß schmeckenden Bestandteil auf.

Die Erfindung beruht auf dem weiteren überraschenden Befund, dass die Aufnahme der Zusammensetzung in Körperzellen durch die Anwesenheit eines süß schmeckenden Bestandteils verbessert bzw. beschleunigt werden kann. Dies gilt insbesondere für den Fall, dass der Bestandteil Ashwagandha oder Ashwagandha-Produkt und/oder der Bestandteil Shankapushpi oder Shankapushpi-Produkt und/oder der Bestandteil Gotu-Kola oder Gotu-Kola-Produkt in Form eines Extraktes, insbesondere wie in der vorangegangenen Beschreibung offenbart, vorliegen/vorliegt. Im Gegensatz hierzu ist die Wirkung von aus dem Stand der Technik bekannten Zusammensetzungen erst nach einigen Tagen oder Wochen nachweisbar. Der mindestens eine süß schmeckende Bestandteil ist vorzugsweise ausgewählt aus der Gruppe bestehend aus Honig, Zucker, Süßstoffe, Zuckeraustauschstoffe und Mischungen von mindestens zwei der vorgenannten süß schmeckenden Bestandteile.

Bei dem Honig kann es sich bevorzugt um Manukahonig handeln, also um einen von Honigbienen aus dem Blütennektar der Südseemyrte (Manuka) erzeugten Honig.

Bei dem Zucker handelt es sich vorzugsweise um Saccharose und/oder Glukose.

Die Süßstoffe können insbesondere ausgewählt sein aus der Gruppe bestehend aus Acesulfam (E950), Advantam (E969), Aspartam (E951), Aspartam-Acesulfam-Salz (E962), Cyclamat (E952), Neohesperidin-Dihydrochalkon (E959), Neotham (E961), Saccharin (E954), Sucralose (E955), Steviosid (E960), Thaumatin (E957), Alitam, Brazzein, Dulcin, Hernandulcin, Lugdunam, Monellin, Mogrosid, Pentadin, Sucrononsäure, Glycyrrhizin, 5-Nitro-2-Propoxyanilin und Mischungen von mindestens zwei der vorgenannten Süßstoffe.

Bei den Zuckeraustauschstoffen handelt es sich vorzugsweise um Polyole, insbesondere Zuckeralkohole. Die Zuckeralkohole können insbesondere ausgewählt sein aus der Gruppe bestehend aus Sorbit (E420), Mannit (E421), Isomalt (E953), Maltit (E965), Lactit (E966), Xylit (E967), Erythrit (E968), Fructose, Inolin, Isomaltulose, Maissirup, Oligofructose, Stärkehydrolysat, Trehalose, Trehalulose und Mischungen von wenigstens zwei der vorgenannten Zuckeraustauschstoffe.

Handelt es sich bei der Zusammensetzung beispielsweise um ein Getränk oder ist die Zusammensetzung beispielsweise Bestandteil eines Getränks, kann es bevorzugt sein, wenn die Zusammensetzung oder das Getränk pro Darreichungsportion oder Darreichungsform 100 mg bis 17.000 mg, insbesondere 10.000 mg bis 16.000 mg von dem mindestens einen süß schmeckenden Bestandteil aufweist.

Insbesondere kann der mindestens eine süß schmeckende Bestandteil einen Anteil von 0,2 Gew.-% bis 34 Gew.-%, insbesondere 1 Gew.-% bis 34 Gew.-%, vorzugsweise 2 Gew.-% bis 32 Gew.-%, aufweisen, bezogen auf das Gesamtgewicht der Zusammensetzung.

In weiterer Ausgestaltung der Erfindung weist die Zusammensetzung ferner mindestens ein Vitamin auf. Bei dem mindestens einen Vitamin kann es sich insbesondere um mindestens ein B-Vitamin handeln. Das mindestens eine B-Vitamin ist vorzugsweise ausgewählt aus der Gruppe bestehend aus Thiamin, Riboflavin, Niacin, Pantothensäure, Pyridoxin, Biotin, Folsäure, Cobalamin und Mischungen von mindestens zwei der vorgenannten B-Vitamine.

Bevorzugt weist die Zusammensetzung pro Darreichungsportion oder Darreichungsform 0,5 mg bis 5 mg, insbesondere 1 mg bis 2 mg, von dem mindestens ein Vitamin, insbesondere mindestens einen B-Vitamin, auf.

Insbesondere kann das mindestens eine Vitamin, insbesondere mindestens eine B-Vitamin, einen Anteil von 0,001 Gew.-% bis 0,01 Gew.-%, insbesondere 0,002 Gew.-% bis 0,006 Gew.- %, vorzugsweise 0,002 Gew.-% bis 0,004 Gew.-%, aufweisen, bezogen auf das Gesamtgewicht der Zusammensetzung.

Die Zusammensetzung kann ferner mindestens einen Alkohol, mindestens eine Fettsäure, mindestens ein Fett oder eine Mischung von mindestens zwei der vorgenannten Verbindungen aufweisen. Überraschenderweise stellte sich heraus, dass auch Alkohole und/oder Fettsäuren und/oder Fette die Wirkungsweise der Zusammensetzung beschleunigen können.

Bei dem mindestens einen Alkohol kann es sich insbesondere um Ethanol handeln.

Bei der mindestens einen Fettsäure kann es sich beispielsweise um Palmitoleinsäure, Ölsäure, Gadoleinsäure, Cetoleinsäure, Erucasäure, Linolsäure, Alpha-Linolensäure, Gamma-Linolensäure, Arachidonsäure, Eicosapentaensäure, Docosadiensäure, Docosatetraensäure, Docosapentaensäure, Docosahexaensäure, Tetracosahexaensäure oder Mischungen von mindestens zwei der vorgenannten Fettsäuren handeln.

In weiterer Ausgestaltung der Erfindung weist die Zusammensetzung ferner mindestens einen Saft, insbesondere mindestens einen Fruchtsaft und/oder mindestens einen Gemüsesaft, vorzugsweise mindestens einen Fruchtsaft, auf.

Unter dem Ausdruck "Fruchtsaft" soll im Sinne der vorliegenden Erfindung in Übereinstimmung mit der Richtlinie 2001/211/EG ein gärfähiges, jedoch nicht gegorenes, aus dem genießbaren Teil gesunder und reifer Früchte einer oder mehrerer Fruchtarten gewonnenes Erzeugnis verstanden werden, das die dafür charakteristische Farbe, das dafür charakteristische Aroma und den dafür charakteristischen Geschmack aufweist.

Der mindestens eine Fruchtsaft kann insbesondere ausgewählt sein aus der Gruppe bestehend aus Traubensaft, Apfelsaft, Orangensaft, Himbeersaft, Johannisbeerensaft, Bananensaft, Maracujasaft und Mischungen von mindestens zwei der vorgenannten Fruchtsäfte.

Unter dem Ausdruck "Gemüsesaft" soll im Sinne der vorliegenden Erfindung ein unverdünntes, zum unmittelbaren Verzehr bestimmtes, gärfähiges und unvergorenes oder milchsauer vergorenes, flüssiges Erzeugnis aus Gemüse verstanden werden.

Alternativ oder in Kombination kann die Zusammensetzung ferner Wasser, insbesondere Trinkwasser, aufweisen.

Ferner kann die Zusammensetzung mindestens einen weiteren Bestandteil, ausgewählt aus der Gruppe bestehend aus Pflanzen-Präparate, Bindemittel, Verdickungsmittel, Konservierungsmittel, Magnesiumcitrate wie Trimagnesiumdicitrat, Bambus und Mischungen von mindestens zwei der vorgenannten Bestandteile, aufweisen.

Die Pflanzen-Präparate können beispielsweise ausgewählt aus der Gruppe bestehend aus Ginkgo-Biloba-Präparat, Grüntee-Präparat, Tulsi-Präparat, Passionsblumen-Präparat, Shatavari-Präparat, Lavendel-Blüten-Präparat, Ajowan-Präparat, Bockshornklee-Samen-Präparat, Curcuma-Präparat, Fenchelsamen-Präparat, Gewürznelken-Präparat, Ingwer-Präparat, Langer Pfeffer-Präparat, Kreuzkümmel-Samen-Präparat und Mischungen von mindestens zwei der vorgenannten Pflanzen-Präparate.

Vorzugsweise handelt es sich bei den Pflanzen-Präparaten um Pflanzenextrakte, insbesondere ausgewählt aus der Gruppe bestehend aus Ginkgo-Biloba-Extrakt, Grüntee-Extrakt, Tulsi-Extrakt, Passionsblumen-Extrakt, Shatavari-Extrakt, Lavendel-Blüten-Extrakt, Ajowan-Extrakt, Bockshornklee-Samen-Extrakt, Curcuma-Extrakt, Fenchelsamen-Extrakt, Gewürznelken-Extrakt, Ingwer-Extrakt, Langer Pfeffer-Extrakt, Kreuzkümmel-Samen-Extrakt und Mischungen von mindestens zwei der vorgenannten Pflanzen-Extrakte.

Alternativ kann es sich bei den Pflanzenpräparaten um ein Pflanzenpulver, insbesondere um ein Pulver aus Ginkgo-Biloba, Grüntee, Tulsi, Passionsblumen, Shatavari, Lavendel-Blüten, Ajowan, Bockshornklee-Samen, Curcuma, Fenchelsamen, Gewürznelken, Ingwer, Langer Pfeffer (Piper longum), Kreuzkümmel-Samen oder um Mischungen von mindestens zwei der vorgenannten Pflanzenpulver handeln.

Bei dem Verdickungsmittel kann es sich beispielsweise um Guarkernmehl handeln.

Bei dem Konservierungsmittel kann es sich beispielsweise um Kaliumsorbat handeln.

Die Zusammensetzung liegt vorzugsweise in flüssiger Form, insbesondere als Getränk, bevorzugt Getränkeshot oder Smoothie, vor.

Alternativ kann die Zusammensetzung in fester Form, insbesondere in Form von Tabletten, Kapseln, eines Pulvers oder eines Granulats, vorliegen.

Weiter kann es sich bei der Zusammensetzung um eine nichtmedizinische Zusammensetzung handeln. Alternativ kann es sich bei der Zusammensetzung um eine medizinische, insbesondere pharmazeutische, Zusammensetzung handeln.

Gemäß einem zweiten Aspekt betrifft die Erfindung ein Nahrungs- oder Genussmittel, insbesondere Nahrungsergänzungsmittel.

Bei dem Nahrungs- oder Genussmittel, insbesondere Nahrungsergänzungsmittel, handelt es sich vorzugsweise um ein Getränk, insbesondere Getränkeshot oder Smoothie.

Das Nahrungs- oder Genussmittel, insbesondere Nahrungsergänzungsmittel, weist eine Zusammensetzung gemäß erstem Erfindungsaspekt auf oder besteht aus einer Zusammensetzung gemäß erstem Erfindungsaspekt.

Bezüglich weiterer Merkmale und Vorteile des Nahrungs- oder Genussmittels wird zur Vermeidung von Wiederholungen vollständig auf die im Rahmen des ersten Erfindungsaspekts gemachten Ausführungen Bezug genommen. Die dort insbesondere in Bezug auf die Zusammensetzung beschriebenen Merkmale und Vorteile gelten sinngemäß auch für das Nahrungs- oder Genussmittel gemäß zweitem Erfindungsaspekt.

Gemäß einem dritten Aspekt betrifft die Erfindung die Verwendung einer Zusammensetzung gemäß erstem Erfindungsaspekt als Nahrungs- oder Genussmittel, insbesondere Nahrungsergänzungsmittel, vorzugsweise Getränk wie Getränkeshot oder Smoothie.

Bezüglich weiterer Merkmale und Vorteile der Zusammensetzung sowie des Nahrungs- oder Genussmittels wird zur Vermeidung von Wiederholungen vollständig auf die im Rahmen der bisherigen Beschreibung, d. h. auf die im Rahmen des ersten und zweiten Erfindungsaspekts, gemachten Ausführungen Bezug genommen. Die dort insbesondere in Bezug auf die Zusammensetzung sowie das Nahrungs- oder Genussmittel beschriebenen Merkmale und Vorteile gelten sinngemäß auch für die Verwendung gemäß drittem Erfindungsaspekt.

Gemäß einem vierten Aspekt betrifft die Erfindung ein Medikament oder Arzneimittel, das eine Zusammensetzung gemäß erstem Erfindungsaspekt aufweist oder aus einer Zusammensetzung gemäß erstem Erfindungsaspekt besteht.

Bezüglich weiterer Merkmale und Vorteile des Medikaments oder Arzneimittels wird zur Vermeidung von Wiederholungen vollständig auf die im Rahmen des ersten Erfindungsaspekts gemachten Ausführungen Bezug genommen. Die dort insbesondere in Bezug auf die Zusammensetzung beschriebenen Merkmale und Vorteile gelten sinngemäß auch für das Medikament oder Arzneimittel gemäß viertem Erfindungsaspekt.

Weitere Merkmale und Vorteile der Erfindung ergeben sich aus der nachfolgenden Beschreibung von bevorzugten Ausführungsformen in Form von Beispielen. Dabei können einzelne Merkmale jeweils für sich alleine oder in Kombination miteinander verwirklicht sein. Die nachfolgend beschriebenen Beispiele dienen lediglich der weiteren Erläuterung der Erfindung, ohne die Erfindung auf den Offenbarungsgehalt der Beispiele zu beschränken.

### BEISPIELTEIL

Die unten stehende Rezeptur 1 gibt die Bestandteile einer Ausführungsform einer erfindungsgemäßen Zusammensetzung wieder:

| **Rezeptur 1** | |
|---|---|
| **Zutat** | **Anteil in mg** |
| Ashwagandha Extrakt (14:1) | 200 |
| Cordyceps militaris Extrakt | 240 |
| Ginkgo Biloba Extrakt | 60 |
| Gotu Kola | 200 |
| Grüntee Extrakt | 1000 |
| Gurana Extrakt (20% Koffein) | 750 |
| Manukahonig 400 MGO | 1500 |
| Kaliumsorbat | 50 |
| Kalmus Extrakt (Vacha) | 50 |
| Klamath-Algen Pulver | 800 |
| L-Theanin | 380 |
| Shankapushpi Extrakt | 150 |
| Shilajit | 500 |
| Traubensaft | 43968.6 |
| Tulsi Extrakt | 50 |
| Guarkernmehl (Verdickungsmittel) | 100 |
| Vitamin B6 | 1.4 |
| Gesamtmenge | 50000 |

Die unten stehende **Rezeptur 2** gibt die Bestandteile einer weiteren Ausführungsform einer erfindungsgemäßen Zusammensetzung wieder:

| **Rezeptur 2** | |
|---|---|
| **Zutat** | **Anteil in mg** |
| Ashwagandha Extrakt | 150 |
| Gotu-Kola | 150 |
| Manukahonig 400 MGO | 1500 |
| Kaliumsorbat | 50 |
| Kalmus Extrakt (Vacha)) | 50 |
| Klamath Algen Pulver | 800 |
| L-Theanin | 1000 |
| Passionsblumen Extrakt | 100 |
| Shankapushpi Extrakt | 150 |
| Shatavari Extrakt | 500 |
| Shilajit | 150 |
| Traubensaft | 45248.6 |
| Tulsi Extrakt | 50 |
| Guarkernmehl (Verdickungsmittel) | 100 |
| Vitamin B6 | 1.4 |
| Gesamtmenge | 50000 |

Die untenstehende **Rezeptur 3** gibt die Bestandteile einer weiteren Ausführungsform einer erfindungsgemäßen Zusammensetzung wieder:

| **Rezeptur 3** | |
|---|---|
| **Zutat** | **Anteil in mg** |
| Ashwagandha Extrakt | 300 |
| Gotu Kola | 200 |
| Griffonia Samen Extract (98% L-5-Hydroxytryptophan) | 200 |
| Manukahonig 400 MGO | 1500 |
| Kaliumsorbat | 50 |
| L-Tryptophan | 200 |
| Lavendel-Blüten Extrakt | 50 |
| L-Theanin | 500 |
| Magnesium Tri-Magnesiumdicitrat | 250 |
| Melatonin | 1 |
| Passionsblume | 50 |
| Puffer Aromatisierung | 46047.6 |
| Shankapushpi-Extrakt | 500 |
| Tulsi Extrakt | 50 |
| Guarkernmehl (Verdickungsmittel) | 100 |
| Vitamin B6 | 1.4 |
| Gesamtmenge | 50000 |

## Patentansprüche

1. Zusammensetzung, aufweisend nachfolgende Bestandteile:
- Ashwagandha oder ein Ashwagandha-Produkt,
- Shankapushpi oder ein Shankapushpi-Produkt und
- Gotu-Kola oder ein Gotu-Kola-Produkt.

2. Zusammensetzung nach Anspruch 1, **dadurch gekennzeichnet, dass** die Zusammensetzung ferner nachfolgenden Bestandteil aufweist:
- L-Theanin.

3. Zusammensetzung nach Anspruch 2, **dadurch gekennzeichnet, dass** die Zusammensetzung pro Darreichungsportion 50 mg bis 1000 mg, insbesondere 380 mg bis 1000 mg, von dem Bestandteil L-Theanin aufweist.

4. Zusammensetzung nach einem der vorhergehenden Ansprüche, **dadurch gekennzeichnet, dass** es sich bei dem Ashwagandha-Produkt um einen Extrakt, insbesondere Wurzel-, Rinde-, Blätter-, Stängel-, Blüten-, Samen-, Früchte- oder Beerenextrakt, von Ashwagandha handelt.

5. Zusammensetzung nach Anspruch 4, **dadurch gekennzeichnet, dass** der Extrakt, insbesondere Wurzel-, Blätter-, Stängel- oder Früchteextrakt, von Ashwagandha mindestens einen Inhaltsstoff, ausgewählt aus der Gruppe bestehend aus Withanolide, Withanolidglykoside, Saponine, Sitoindoside, Alkaloide, Steroidlactone, Tropanole, Cuscohygrin, Choline, Fettsäuren, Oligosacccharide, insbesondere Oligosaccharide mit einem relativen Molekulargewicht < 2000, Aminosäuren und Mischungen von mindestens zwei der vorgenannten Inhaltsstoffe, aufweist.

6. Zusammensetzung nach einem der vorhergehenden Ansprüche, **dadurch gekennzeichnet, dass** die Zusammensetzung pro Darreichungsportion 50 mg bis 400 mg, insbesondere 50 mg bis 300 mg, von dem Bestandteil Ashwagandha oder Ashwagandha-Produkt aufweist.

7. Zusammensetzung nach einem der vorhergehenden Ansprüche, **dadurch gekennzeichnet, dass** es sich bei dem Shankapushpi-Produkt um einen Extrakt, insbesondere Wurzel-, Rinde-, Blätter-, Stängel-, Blüten-, Samen-, Früchte- oder Beerenextrakt, von Shankapushpi handelt.

8. Zusammensetzung nach Anspruch 7, **dadurch gekennzeichnet, dass** der Extrakt, insbesondere Wurzel-, Rinde-, Blätter-, Stängel-, Blüten-, Samen-, Früchte- oder Beerenextrakt, von Shankapushpi mindestens einen Inhaltsstoff, ausgewählt ist aus der Gruppe bestehend aus Kämpferol, β-Sitosterol, N-Hexacosanol, Hydroxyzimtsäure und Mischungen von mindestens zwei der vorgenannten Inhaltsstoffe, aufweist.

9. Zusammensetzung nach einem der vorhergehenden Ansprüche, **dadurch gekennzeichnet, dass** die Zusammensetzung pro Darreichungsportion 100 mg bis 600 mg, insbesondere 150 mg bis 500 mg, von dem Bestandteil Shankapushpi oder Shankapushpi-Produkt aufweist.

10. Zusammensetzung nach einem der vorhergehenden Ansprüche, **dadurch gekennzeichnet, dass** es sich bei dem Gotu-Kola-Produkt um einen Extrakt, insbesondere Wurzel-, Rinde-, Blätter-, Stängel-, Blüten-, Samen-, Früchte- oder Beerenextrakt, von Gotu-Kola handelt.

11. Zusammensetzung nach einem der vorhergehenden Ansprüche, **dadurch gekennzeichnet, dass** der Extrakt, insbesondere Wurzel-, Blätter-, Stängel- oder Früchteextrakt, von Gotu-Kola mindestens einen Inhaltsstoff, ausgewählt ist aus der Gruppe bestehend aus Triterpenoide, insbesondere Asiaticoside, Asiatsäure oder Madecassische Säure, Flavonolglykoside, Alkaloide, insbesondere Hydrocotylin, Caryophyllen, Cymen, Germacren, Pinen, und Mischungen von mindestens zwei der vorgenannten Inhaltsstoffe, aufweist.

12. Zusammensetzung nach einem der vorhergehenden Ansprüche, **dadurch gekennzeichnet, dass** die Zusammensetzung pro Darreichungsportion 50 mg bis 300 mg, insbesondere 100 mg bis 200 mg, von dem Bestandteil Gotu-Kola oder Gotu-Kola-Produkt aufweist.

13. Zusammensetzung nach einem der vorhergehenden Ansprüche, **dadurch gekennzeichnet, dass** die Zusammensetzung ferner nachfolgende Bestandteile aufweist:
- Klamath-Algen oder ein Klamath-Algen-Produkt,
- Kalmus oder ein Kalmus-Produkt
und
- Shilajit oder ein Shilajit-Produkt.

14. Zusammensetzung nach einem der vorhergehenden Ansprüche, insbesondere nach Anspruch 13, **dadurch gekennzeichnet, dass** die Zusammensetzung ferner nachfolgende Bestandteile aufweist:
- Koffein und/oder einen Stoff mit koffeinähnlicher Wirkung und/oder ein koffeinhaltiges Produkt
und
- Cordyceps oder ein Cordyceps-Produkt.

15. Zusammensetzung nach einem der vorhergehenden Ansprüche, insbesondere nach einem der Ansprüche 1 bis 12, **dadurch gekennzeichnet, dass** die Zusammensetzung ferner nachfolgende Bestandteile aufweist:
- Melatonin,
- L- Tryptophan und
- 5-Hydroxytryptophan und/oder ein 5-hydroxytryptophanhaltiges Produkt.

16. Zusammensetzung nach einem der vorhergehenden Ansprüche, **dadurch gekennzeichnet, dass** die Zusammensetzung ferner mindestens einen süß schmeckenden Bestandteil, insbesondere ausgewählt aus der Gruppe bestehend aus Honig, Zucker, Süßstoffe, Zuckeraustauschstoffe und Mischungen von mindestens zwei der vorgenannten süß schmeckenden Bestandteile aufweist.

17. Zusammensetzung nach einem der vorhergehenden Ansprüche, **dadurch gekennzeichnet, dass** die Zusammensetzung ferner mindestens ein Vitamin, insbesondere mindestens ein B-Vitamin, vorzugsweise ausgewählt aus der Gruppe bestehend aus Thiamin, Riboflavin, Niacin, Pantothensäure, Pyridoxin, Biotin, Folsäure, Cobalamin und Mischungen von mindestens zwei der vorgenannten B-Vitamine, aufweist.

18. Zusammensetzung nach einem der vorhergehenden Ansprüche, **dadurch gekennzeichnet, dass** die Zusammensetzung ferner mindestens einen Saft, vorzugsweise Fruchtsaft, insbesondere ausgewählt aus der Gruppe bestehend aus Traubensaft, Apfelsaft, Orangensaft, Himbeersaft, Johannisbeerensaft, Bananensaft, Maracujasaft und Mischungen von mindestens zwei der vorgenannten Säfte, aufweist.

19. Nahrungs- oder Genussmittel, insbesondere Getränk, aufweisend oder bestehend aus einer Zusammensetzung nach einem der vorhergehenden Ansprüche.
